# EUROPEAN PATENT APPLICATION

(11) **EP 4 086 916 A1**
(43) Date of publication of application: **09.11.2022**
(21) Application number: 21171851.5
(22) Date of filing: 03.05.2021
(51) Int. Cl.: G16H 30/20, G16H 50/20, G06K 9/00, G06T 7/20, A61B 5/16

(54) **PATIENT POSITIONING ADAPTIVE GUIDANCE SYSTEM**

(71) Applicant: Koninklijke Philips N.V., 5656 AG Eindhoven (NL)
(72) Inventor: DJAJADININGRAT, Johan Partomo, 5656 AE Eindhoven (NL); TALGORN, lise Claude Valentine, 5656 AE Eindhoven (NL); LAUTE, Niels, 5656 AE Eindhoven (NL); KNOESTER, Jaap, 5656 AE Eindhoven (NL); LEE, Hyelin, 5656 AE Eindhoven (NL); BOSMAN, Reinoud, 5656 AE Eindhoven (NL)
(74) Representative: Philips Intellectual Property & Standards

(57) **Abstract**

The present invention relates to a patient positioning adaptive guidance system (10), comprising: a medical image acquisition unit (20); at least one communication unit (30); at least one camera (40); and a processing unit (50). The medical image acquisition unit is configured to acquire a medical image of a patient. The processing unit is configured to control the at least one communication unit to provide body positioning information to the patient prior to acquisition of the medical image. One or more cameras of the at least one camera is configured to acquire position and movement image data of the patient associated with the provision of the body position information to the patient. The processing unit is configured to determine if the patient has not reached a required position due to a physical or cognitive limitation, the determination comprising utilisation of the body positioning information and the position and movement image data of the patient, wherein a determination is made that the patient has not reached the required position due to a physical limitation based on a determination that the patient has moved in a correct direction, and wherein a determination is made that the patient has not reached the required position due to a cognitive limitation based on a determination that the patient has moved in an incorrect direction or has not undertaken a substantive movement. The processing unit is configured to adapt the body positioning information in at least one first manner based on a determination that the patient has not reached the required position due to a physical limitation. The processing unit is configured to adapt the body positioning information in at least one second manner based on a determination that the patient has not reached the required position due to a cognitive limitation.

## Description

### FIELD OF THE INVENTION

The present invention relates to a patient positioning adaptive guidance system, a patient positioning adaptive guidance method, as well as to a computer program element and a computer readable medium.

### BACKGROUND OF THE INVENTION

For many medical examinations the patient has to adopt a certain position and a certain posture. For example, for an MR scan, the patient may need to put her hands above her head. For a knee examination, she may need to bend her knee. For a chest X-ray, the patient may need to put her hands on her back and curl her shoulder blades forward. Currently, there are experienced nurses and technicians who explain to the patient what to do and may push the patient into the right position and posture.

A challenge in providing guidance is that patients differ, both from a physical and a cognitive perspective. Physically, some patients can easily adopt the required position whilst others may not be able to reach the required position, for example because they are elderly, have arthritis, are an outlier in terms of body morphology (e.g. have a large belly or breasts) or have some other mobility impairment. From a cognitive point of view, some people may (initially) have troubles to understand the movement guidance whilst others may understand it straight away. This depends on the familiarity of the people with the guidance media, their cognitive abilities, and their ability to relate a movement guidance to their body movement (i.e. a dancer will find it easier).

An experienced nurse will typically correctly analyse the situation. If the patient is not physically capable of achieving the suggested posture the nurse will not keep pushing for the ideal position but settle for the best achievable position. If the patient does not understand, the nurse will re-explain or explain in a different way.

However, due to the rising costs of healthcare, and the trend to bring diagnostic imaging to delocalised health centres with less specialised staff, there is a movement towards using less trained staff who may lack the knowledge, skills and experience to help patients adopt the right posture. There is even discussions relating to undertaking such medical examinations completely automatically, with no medical staff present.

However, currently problems can occur, when only inexperienced staff are present, where incorrect image data is acquired and there is currently no ability to undertake such examinations in an automated manner.

There is a need to resolve these issues.

### SUMMARY OF THE INVENTION

'It would be advantageous to have an improved means of positioning a patient for a medical examination. The object of the present invention is solved with the subject matter of the independent claims, wherein further embodiments are incorporated in the dependent claims. It should be noted that the following described aspects and examples of the invention apply to the patient positioning adaptive guidance system, and also to the patient positioning adaptive guidance method as well as to the computer program element and the computer readable medium.

'In a first aspect, there is provided a patient positioning adaptive guidance system, comprising:
- a medical image acquisition unit;
- at least one communication unit;
- at least one camera; and
- a processing unit.

'The medical image acquisition unit is configured to acquire a medical image of a patient. The processing unit is configured to control the at least one communication unit to provide body positioning information to the patient prior to acquisition of the medical image. One or more cameras of the at least one camera is configured to acquire position and movement image data of the patient associated with the provision of the body position information to the patient. The processing unit is configured to determine if the patient has not reached a required position due to a physical or cognitive limitation, the determination comprising utilisation of the body positioning information and the position and movement image data of the patient. A determination is made that the patient has not reached the required position due to a physical limitation based on a determination that the patient has moved in a correct direction. A determination is made that the patient has not reached the required position due to a cognitive limitation based on a determination that the patient has moved in an incorrect direction or has not undertaken a substantive movement. The processing unit is configured to adapt the body positioning information in at least one first manner based on a determination that the patient has not reached the required position due to a physical limitation. The processing unit is configured to adapt the body positioning information in at least one second manner based on a determination that the patient has not reached the required position due to a cognitive limitation.

'In other words, a system analyses the response behaviour through computer vision, that could for example use a depth camera with skeleton recognition for movement and position analysis and through camera-based facial analysis for expression analysis. Classification of the user's mistake in term of cognitive or physical limitation is undertaken, and a determination is made that there is a physical limitation if the patient's initial movement direction is correct, whilst a determination is made that there is a cognitive limitation if the patient does not move, or moves in a completely different direction than the guidance instruction. Then, if the limitation is physical, guidance is adapted to match the patient's physical abilities, and if the limitation is cognitive, the presented guidance is adapted through for example presenting imagery in the form of a character of avatar the body of which is morphed to resemble more closely the body of the patient, taking into account patient gender, height, and weight etc. 'In this way, a patient can be automatically guided to the correct or optimum position for a medical image to be acquired based on the behaviour of the user, that takes into account physical or cognitive limitations of the patient.

Furthermore, the new system provides for time saving. This is because it is no longer required for the nurse to walk back and forth to the control room to make adjustments to the patient's posture. The self-guided system will do that for her/him. For example, such an exam can normally take 60 seconds (and they do a lot of everyday), but with elderly it might take a few minutes doing the adjustments. The new system then speeds this up significantly, and is a big timesaver for those situations.

In an example, one or more cameras of the at least one camera is configured to acquire facial image data of the patient associated with the provision of the body position information to the patient. The determination if the patient has not reached a required position due to a physical or cognitive limitation can then comprise utilization of the facial image data of the patient associated with the provision of the body position information to the patient.

In an example, the processing unit is configured to determine if the facial image data indicates that the patient is in pain or determine if the facial image data indicates that the patient is confused. The wherein determination if the patient has not reached a required position due to a physical or cognitive limitation can then comprise utilization of the determination if the patient is in pain or is confused.

Thus, if the patient has moved in a correct direction, but not reached the correct position, and facial analysis indicates that they are in pain, then the guidance could be to move to a different end position through a different movement direction, or accept that the patient cannot move to the first indicated correct position and accept that an end position as close as possible to the correct end position should be accepted, and the movement guidance provided to the patient can be adapted accordingly. Also, if the patient has not moved at all, or has moved in a wrong direction and at the same time appears to look confused, then the guidance provided to the patient can take into account this, and be adapted to enable the patient better to understand the required movement and the required end correct position required.

In an example, the body positioning information comprises visual and/or audio information and/or haptic feedback.

In an example, the processing unit is configured to control the at least one communication unit to provide the body positioning information in the form of information relating to movement from a start position to the required position.

In an example, the information relating to movement from the start position to the required position is in the form of at least one repeatable loop.

In an example, the information relating to movement from the start position to the required position is in the form of a plurality of repeatable loops, each loop relating to a different section of movement from the start position to the required position.

In this manner, a required movement of the body from an initial position to an end position can be sub-divided into video loops relating to different portions of the overall movement. Each loop can be played in turn as the patient undertakes that part of the overall movement, and if they are having difficulty going through a segment of the overall movement that particular video loop can be replayed. Then when they have reached the end of a movement section, the next video loop can be played etc. Here video is used in a generic manner and can relate to an animation that can be morphed or adapted or relate to a fixed video segment.

In an example, the at least one first manner comprises providing encouragement to the patient to move to the required position.

To put this another way, if the patient has almost reached a correct position, an audio and/or visual presentation can be provided to the patient to for example "please rotate your body just a little bit more to the left". Haptic information, such as a series of pulses can also be provided to encourage the patient to move, where for example a temporal spacing between pulses becomes less as the correct position is approached. The pulses could then stop or change to indicate that the correct position has been reached for example.

In an example, the at least one first manner comprises adapting the body positioning information to movement capabilities of the patient, and/or wherein the at least one first manner comprises adapting the body positioning information to physical characteristics of the patient.

In this manner, it can be established that for some reason, that could for example be that the patient has pain in a joint, the patient cannot move as required and the information provided to the patient be adapted to either help them attain the required position or change the required position.

Also, it can be established that the patient is physically unable to attain the required end position. Image processing could for example establish that a man has a large belly and cannot move to a position as required or that a woman has large breasts and cannot move to a position as required and the movement information be adapted to provide to the patient a best possible position to attain that takes into account the physical characteristics of the patient.

In an example, the at least one second manner comprises changing an Avatar to have a body shape that more closely matches the patient's body shape or changing the Avatar to be more abstract.

In an example, the at least one second manner comprises changing a viewing angle from which body positioning information in terms of visual instructions are presented to the patient, and/or adding at least one enhanced visual element to visual information. The enhanced visual elements can comprise one or more of: text; arrows; highlights.

In an example, the at least one second manner comprises changing a time length of a repeatable loop, changing an order of a plurality of loop segments, changing a speed playback of a repeatable loop.

In an example, the at least one second manner comprises changing the body positioning information from visual information to audio information, or changing the body positioning information from audio information to visual information, or changing the body positioning information from visual information to visual information and audio information, or changing the body positioning information from audio information to visual information and audio information.

In a second aspect, there is provided a patient positioning adaptive guidance method, comprising:
a) controlling by a processing unit at least one communication unit to provide body positioning information prior to acquisition of a medical image of a patient;
b) acquiring by one or more cameras of at least one camera position and movement image data of the patient associated with the provision of the body position information to the patient;
c) determining by the processing unit if the patient has not reached a required position due to a physical or cognitive limitation, the determining comprising utilising the body positioning information and the position and movement image data of the patient, wherein a determination is made that the patient has not reached the required position due to a physical limitation based on a determination that the patient has moved in a correct direction, and wherein a determination is made that the patient has not reached the required position due to a cognitive limitation based on a determination that the patient has moved in an incorrect direction or has not undertaken a substantive movement;
   wherein the method comprises either:
   adapting by the processing unit the body positioning information in at least one first manner based on a determination that the patient has not reached the required position due to a physical limitation; or
   adapting by the processing unit the body positioning information in at least one second manner based on a determination that the patient has not reached the required position due to a cognitive limitation; and
d) after adapting the body positioning information acquiring by a medical image acquisition unit the medical image of the patient.

According to another aspect, there is provided a computer program element controlling one or more of the systems as previously described which, if the computer program element is executed by a processing unit, is adapted to perform the method as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows a schematic set up of an example of a patient positioning adaptive guidance system;
Fig. 2 shows a patient positioning adaptive guidance method; and
Fig. 3 shows three snapshots from loop segments of positioning information.

### DETAILED DESCRIPTION OF EMBODIMENTS

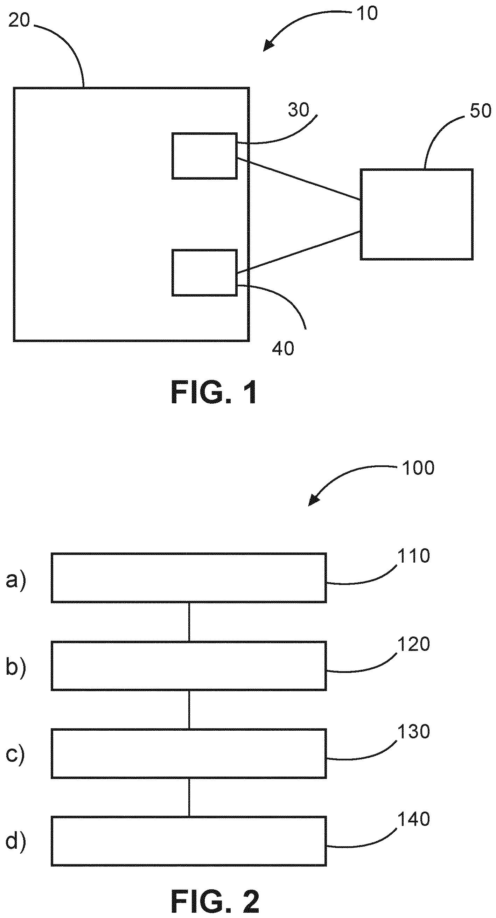

Fig. 1 shows a schematic example of a patient positioning adaptive guidance system 10. The system comprises a medical image acquisition unit 20, at least one communication unit 30, at least one camera 40, and a processing unit 50. The medical image acquisition unit is configured to acquire a medical image of a patient. The processing unit is configured to control the at least one communication unit to provide body positioning information to the patient prior to acquisition of the medical image. One or more cameras of the at least one camera is configured to acquire position and movement image data of the patient associated with the provision of the body position information to the patient. The processing unit is configured to determine if the patient has not reached a required position due to a physical or cognitive limitation, the determination comprising utilisation of the body positioning information and the position and movement image data of the patient. A determination is made that the patient has not reached the required position due to a physical limitation based on a determination that the patient has moved in a correct direction. A determination is made that the patient has not reached the required position due to a cognitive limitation based on a determination that the patient has moved in an incorrect direction or has not undertaken a substantive movement. The processing unit is configured to adapt the body positioning information in at least one first manner based on a determination that the patient has not reached the required position due to a physical limitation. The processing unit is configured to adapt the body positioning information in at least one second manner based on a determination that the patient has not reached the required position due to a cognitive limitation.

According to an example, one or more cameras of the at least one camera is configured to acquire facial image data of the patient associated with the provision of the body position information to the patient. The determination if the patient has not reached a required position due to a physical or cognitive limitation can then comprise utilization of the facial image data of the patient associated with the provision of the body position information to the patient.

According to an example, the processing unit is configured to determine if the facial image data indicates that the patient is in pain or determine if the facial image data indicates that the patient is confused. The wherein determination if the patient has not reached a required position due to a physical or cognitive limitation can then comprise utilization of the determination if the patient is in pain or is confused.

According to an example, the body positioning information comprises visual and/or audio information and/or haptic feedback.

In an example the visual information comprises utilization of an avatar.

In example, the avatar undertakes the required movements the patient must undertake to achieve the required position.

In an example, the visual information comprises a representation of the present position of the patient and a silhouette representing the required position.

In an example, the haptic feedback is provided via a wearable device. In an example, the wearable device is a smart watch.

According to an example, the processing unit is configured to control the at least one communication unit to provide the body positioning information in the form of information relating to movement from a start position to the required position.

According to an example, the information relating to movement from the start position to the required position is in the form of at least one repeatable loop.

According to an example, the information relating to movement from the start position to the required position is in the form of a plurality of repeatable loops, each loop relating to a different section of movement from the start position to the required position.

According to an example, the at least one first manner comprises providing encouragement to the patient to move to the required position.

According to an example, the at least one first manner comprises adapting the body positioning information to movement capabilities of the patient. Alternatively or additionally the at least one first manner comprises adapting the body positioning information to physical characteristics of the patient.

According to an example, the at least one second manner comprises changing an Avatar to have a body shape that more closely matches the patient's body shape or changing the Avatar to be more abstract.

According to an example, the at least one second manner comprises changing a viewing angle from which body positioning information in terms of visual instructions are presented to the patient. Alternatively or additionally the at least one second manner comprises adding at least one enhanced visual element to visual information, and the enhanced visual elements can comprises one or more of: text; arrows; highlights.

According to an example, the at least one second manner comprises changing a time length of a repeatable loop, changing an order of a plurality of loop segments, changing a speed playback of a repeatable loop.

According to an example, the at least one second manner comprises changing the body positioning information from visual information to audio information.

According to an example, the at least one second manner comprises changing the body positioning information from audio information to visual information.

According to an example, the at least one second manner comprises changing the body positioning information from visual information to visual information and audio information.

According to an example, the at least one second manner comprises changing the body positioning information from audio information to visual information and audio information.

In an example, the system comprises at least one stress sensor configured to acquire stress level information from the patient, and wherein the determination if the patient has not reached a required position due to a physical or cognitive limitation comprises utilization of the stress level information.

In an example, the stress level information comprises one or more of: heart beat, perspiration level, skin conductance, breathing rate.

Fig. 2 shows a patient positioning adaptive guidance method 100 in its basic steps. The method comprises:
In a controlling step 110, also termed step a), controlling by a processing unit at least one communication unit to provide body positioning information prior to acquisition of a medical image of a patient;
In an acquiring step 120, also termed step b), acquiring by one or more cameras of at least one camera position and movement image data of the patient associated with the provision of the body position information to the patient;
In a determining step 130, also termed step c), determining by the processing unit if the patient has not reached a required position due to a physical or cognitive limitation, the determining comprising utilising the body positioning information and the position and movement image data of the patient, wherein a determination is made that the patient has not reached the required position due to a physical limitation based on a determination that the patient has moved in a correct direction, and wherein a determination is made that the patient has not reached the required position due to a cognitive limitation based on a determination that the patient has moved in an incorrect direction or has not undertaken a substantive movement;
wherein the method comprises either:
   adapting by the processing unit the body positioning information in at least one first manner based on a determination that the patient has not reached the required position due to a physical limitation; or
   adapting by the processing unit the body positioning information in at least one second manner based on a determination that the patient has not reached the required position due to a cognitive limitation; and
   in an acquiring step 140, also termed step d), after adapting the body positioning information acquiring by a medical image acquisition unit the medical image of the patient.

In an example, the method comprises acquiring by one or more cameras of the at least one camera facial image data of the patient associated with the provision of the body position information to the patient, and wherein the determining if the patient has not reached a required position due to a physical or cognitive limitation comprises utilizing the facial image data of the patient associated with the provision of the body position information to the patient.

In an example, the method comprises determining by the processing unit if the facial image data indicates that the patient is in pain or determining if the facial image data indicates that the patient is confused, and the wherein the determining if the patient has not reached a required position due to a physical or cognitive limitation comprises utilizing the determination if the patient is in pain or is confused.

In an example, the body positioning information comprises visual information and/or audio information and/or haptic feedback.

In an example the visual information comprises an avatar.

In example, the avatar undertakes the required movements the patient must undertake to achieve the required position.

In an example, the haptic feedback is provided via a wearable device. In an example, the wearable device is a smart watch.

In an example, the visual information comprises a representation of the present position of the patient and a silhouette representing the required position.

In an example, the method comprises controlling by the processing unit the at least one communication unit to provide the body positioning information in the form of information relating to movement from a start position to the required position.

In an example, the information relating to movement from the start position to the required position is in the form of at least one repeatable loop.

In an example, the information relating to movement from the start position to the required position is in the form of a plurality of repeatable loops, each loop relating to a different section of movement from the start position to the required position.

In an example, the at least one first manner comprises providing encouragement to the patient to move to the required position.

In an example, the at least one first manner comprises adapting the body positioning information to movement capabilities of the patient, and/or the at least one first manner comprises adapting the body positioning information to physical characteristics of the patient.

In an example, the at least one second manner comprises changing an Avatar to have a body shape that more closely matches the patient's body shape or changing the Avatar to be more abstract.

In an example, the at least one second manner comprises changing a viewing angle from which body positioning information in terms of visual instructions are presented to the patient, and/or adding at least one enhanced visual element to visual information, wherein the enhanced visual elements comprises one or more of: text; arrows; highlights

In an example, the at least one second manner comprises changing a time length of a repeatable loop, changing an order of a plurality of loop segments, changing a speed playback of a repeatable loop.

In an example, the at least one second manner comprises changing the body positioning information from visual information to audio information, or changing the body positioning information from audio information to visual information, or changing the body positioning information from visual information to visual information and audio information, or changing the body positioning information from audio information to visual information and audio information.

In an example, the method comprises acquiring by at least one stress sensor stress level information from the patient, and wherein the determining if the patient has not reached a required position due to a physical or cognitive limitation comprises utilizing the stress level information.

In an example, the stress level information comprises one or more of: heart beat, perspiration level, skin conductance, breathing rate.

Thus, the system and method describes a number of rules so that a processing unit, such as that within a computer, can intelligently adapt the guidance to the movements of the patient. In particular, it provides a way of distinguishing between a patient not being able to adopt the right posture because of physical limitations and because of cognitive or mental limitations.

The system and method helps to instruct the patient what posture to adopt. Such instructions can be visual (e.g. 2D visuals, 3D visuals, animated visuals, video) and/or audible (e.g. spoken or abstract) and/or haptic (e.g. wearable device or one integrated into the stand), and this is enabled because the system/method establishes if the patient is not following the instructions because of a physical or cognitive limitation, and adapts the instructions accordingly.

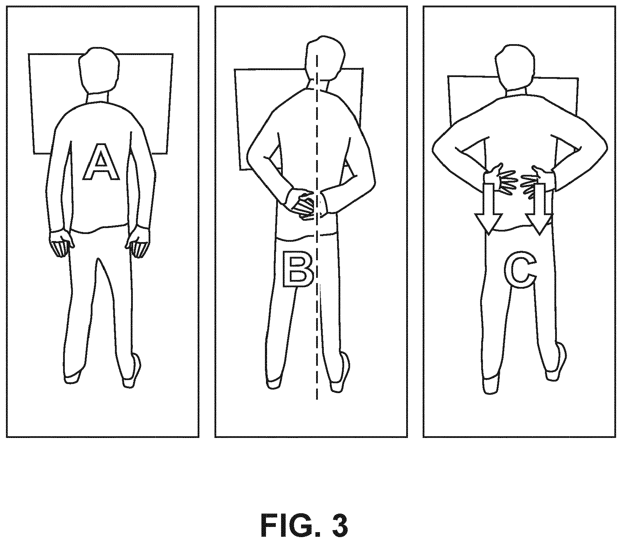

The patient positioning adaptive guidance system and patient positioning adaptive guidance method are explained in specific detail, where reference is made to Fig. 3.

Fig. 3 shows three snapshots from loop segments of positioning information. The inventors designed a guidance concept for a chest X-ray image unit, or other X-ray or MRI or PET image system, based on short loops of 3D animation. In such a loop, an animated human character (e.g. an avatar or 'virtual twin'), augmented with visual instructions such as arrows, shows the movement that is required to move from the patient's current posture into the required posture, together with audible instructions. This is represented in Fig. 3, where a complex movement is 'chopped' up into understandable chunks. Each of the three pictures shown in fig. 3 is in fact an animation loop, where only one snapshot image from that loop is shown. In the first loop segment "A" represents "Put your hands behind your back", in the second loop segment "B" represented "Tilt to the left" and in the third loop segment "C" represented "Move hands down". The loops can be a real-life video, with an actual person performing the movements, of the person can be a 3D animated character of which the movements can be adapted in real-time. Adaptation of the character or avatar in real-time has been shown to aid people who have found it difficult to follow the instructions, for example by making the avatar more abstract (and fun for a child because it can look like a cartoon character) or bay making the avatar loop more like the patient, from imagery of the patient being acquired, in order that the patient can better understand that they have to do what the avatar is doing.

Another guidance principle, not shown, is 'shadow guidance', where the user tries to match its silhouette to a target silhouette. Thus, a target shadow can undertake the required movement and superimposed on what the patient is seeing on a screen is a rendition of the position of their own body, and they move their own body to make the shadow on the screen follow the target shadow.

Thus, a summary of how the system/method operates can be detailed as:
A guidance part of the system shows instructions to a user on the steps to reach a desired target position. The guidance can be visual (2D visuals, 3D visuals, animated visuals, video) and/or audible (e.g. spoken or abstract) and/or haptic (e.g. hardware movements or vibration to actively push or suggest).

A part of the system analyses the response behaviour through computer vision, that can be through using a depth camera with skeleton recognition for movement and position analysis and through camera-based facial analysis for expression analysis. The system can also gather stress data from a wearable sensor such as a Heart rate or skin conductance sensor, or via utilization of audio / speech analysis.

A classification of the user's mistake in term of cognitive or physical limitation is carried out:
Physical if the patient movement's initial direction is correct (similar to the guidance instruction) but the amplitude or the final direction of the movement is incorrect (hence target position is not reached)
   Cognitive if the patient does not move, or moves in a completely different direction than the guidance instruction, or presents signs of perplexity (facial expression recognition) or stress.
   An adaptation of the guidance principle is made
If the limitation is physical, adapt guidance to match the patient physical abilities (detected by computer vision)
**Amplitude of character movement (hence target position)** is adapted to the detected movement amplitude of the patient
**Amplitude of character movement** is adapted to the limiting features of the patient's body (e.g. large belly, breasts).

If the limitation is cognitive, adapt the presented guidance:
**Body morphology of character** is adapted to the body morphology of the patient (gender, height, weight)
**Angle of view** is adapted to the amplitude of the patient's movements. Some movements will be more visible under certain angles and when the patient does not comply, the angle of view may be adapted to better visualize that movement.

**Speed of character movement** is adapted to detected speed of patient e.g. when walking
**Guidance rhythm** is changed e.g. the 3D loops are shorter, or in a different order
**Enhancing visual elements** are added such as text, arrows, highlights
**Visual look is changed** e.g. from a realistic avatar to an abstracted avatar
**Guidance modality** is changed or two modalities are combined (visual, audible, haptic) Thus, an exemplar system can do the following: check if the desired posture is reached. If not, a check is made if the user is trying to move towards this position, by for example measuring body movement). To augment this, the facial expression can be checked to see if the user is either confused where it can be determined that the facial expression represents for example "how would I move towards this position?") or whether the user is expressing frustration where it can be determined that the facial expression represented for example "I can't move to this position due limitation of my body movements".

### Detailed workflow

The following relates to a detailed workflow regarding acquisition of a frontal chest X-ray, but the workflow can apply to different X-ray examinations and different types of examination, such as MR/PET.

The total posture change required is split up into manageable, understandable sub-movements (e.g. the frontal chest x-ray is split up into (i) stand in against of the middle of the wallstand, (ii) put your hands behind your back, (iii) curl your shoulders against the wallstand)

Each of these sub-movements is explained through a looped 3D animation. The loop shows a 3D animated character acting out the required movement, again and again. Body parts and their movement may be visually emphasized through various visual means (e.g. arrows, colour and lighting).

Using computer vision (e.g. a depth camera) the patient's body morphology is analysed.

Using the patient's body morphology, the system adapts the target posture (e.g. in case of patient with a big belly or breast undergoing a chest X-ray, the patient may not be able to move his/her shoulders fully against the detector plate).

Using computer vision (e.g. a depth camera) the patient's response is analysed for compliance with the set example and the speed of compliance.

In this manner a number of behavioural patterns can be distinguished:

### Phase 0 - Cognitive overload/mental confusion:

Patient response: if the patient does not respond, moves in the wrong direction, shows signs of confusion (e.g. erratic movement, facial expression), this means he does not understand the instructions.

### System adaptation:

repeat and/or rephrase the instruction.
animate the virtual camera to show the movement from different angles.
settle for a less optimal but simpler to reach target posture.

### Phase 1 - Capable of carrying out the movement, but not completing it accurately

Patient response: if patient movement's initial direction is correct but the amplitude incorrect, this may be caused by a misunderstanding of the amplitude (i.e. the patient thinks he is moving far enough but is in fact not).

### System adaptation:

Encourage the patient to move further
Zoom in on the last part of the animation loop

### Phase 3 - Physically unable to carry out the movement:

Patient response: if patient movement's initial direction is correct but the amplitude remains incorrect, **even after repeated encouragement of phase 2,** this probably means that the patient is not physically capable of reaching the target position. In addition, the patient's facial expression may be analyzed for pain to see whether the patient is straining himself.

### System adaptation:

Switch to an alternative target position (e.g. for a chest x-ray, patients may put their hands behind their back but if this doesn't work for them they may 'hug' the wallstand)

Settle for the right movement but with less amplitude.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A patient positioning adaptive guidance system (10), comprising:
- a medical image acquisition unit (20);
- at least one communication unit (30);
- at least one camera (40); and
- a processing unit (50);
wherein the medical image acquisition unit is configured to acquire a medical image of a patient;
wherein the processing unit is configured to control the at least one communication unit to provide body positioning information to the patient prior to acquisition of the medical image;
wherein one or more cameras of the at least one camera is configured to acquire position and movement image data of the patient associated with the provision of the body position information to the patient;
wherein the processing unit is configured to determine if the patient has not reached a required position due to a physical or cognitive limitation, the determination comprising utilisation of the body positioning information and the position and movement image data of the patient, wherein a determination is made that the patient has not reached the required position due to a physical limitation based on a determination that the patient has moved in a correct direction, and wherein a determination is made that the patient has not reached the required position due to a cognitive limitation based on a determination that the patient has moved in an incorrect direction or has not undertaken a substantive movement;
wherein the processing unit is configured to adapt the body positioning information in at least one first manner based on a determination that the patient has not reached the required position due to a physical limitation; and
wherein the processing unit is configured to adapt the body positioning information in at least one second manner based on a determination that the patient has not reached the required position due to a cognitive limitation.

2. System according to claim 1, wherein one or more cameras of the at least one camera is configured to acquire facial image data of the patient associated with the provision of the body position information to the patient, and wherein the determination if the patient has not reached a required position due to a physical or cognitive limitation comprises utilization of the facial image data of the patient associated with the provision of the body position information to the patient.

3. System according to claim 2, wherein the processing unit is configured to determine if the facial image data indicates that the patient is in pain or determine if the facial image data indicates that the patient is confused, and the wherein determination if the patient has not reached a required position due to a physical or cognitive limitation comprises utilization of the determination if the patient is in pain or is confused.

4. System according to any of claims 1-3, wherein the body positioning information comprises visual and/or audio information and/or haptic feedback.

5. System according to any of claims 1-4, wherein the processing unit is configured to control the at least one communication unit to provide the body positioning information in the form of information relating to movement from a start position to the required position.

6. System according to claim 5, wherein the information relating to movement from the start position to the required position is in the form of at least one repeatable loop.

7. System according to claim 6, wherein the information relating to movement from the start position to the required position is in the form of a plurality of repeatable loops, each loop relating to a different section of movement from the start position to the required position.

8. System according to any of claims 1-7, wherein the at least one first manner comprises providing encouragement to the patient to move to the required position.

9. System according to any of claims 1-8, wherein the at least one first manner comprises adapting the body positioning information to movement capabilities of the patient, and/or wherein the at least one first manner comprises adapting the body positioning information to physical characteristics of the patient.

10. System according to any of claims 1-9, wherein the at least one second manner comprises changing an Avatar to have a body shape that more closely matches the patient's body shape or changing the Avatar to be more abstract.

11. System according to any of claims 1-10, wherein the at least one second manner comprises changing a viewing angle from which body positioning information in terms of visual instructions are presented to the patient, and/or adding at least one enhanced visual element to visual information, wherein the enhanced visual elements comprises one or more of: text; arrows; highlights.

12. System according to any of claims 6-7 or claims 8-11 when dependent upon any of claims 6-7, wherein the at least one second manner comprises changing a time length of a repeatable loop, changing an order of a plurality of loop segments, changing a speed playback of a repeatable loop.

13. System according to any of claims 1-12, wherein the at least one second manner comprises changing the body positioning information from visual information to audio information, or changing the body positioning information from audio information to visual information, or changing the body positioning information from visual information to visual information and audio information, or changing the body positioning information from audio information to visual information and audio information.

14. A patient positioning adaptive guidance method (100), comprising:
a) controlling (110) by a processing unit at least one communication unit to provide body positioning information prior to acquisition of a medical image of a patient;
b) acquiring (120) by one or more cameras of at least one camera position and movement image data of the patient associated with the provision of the body position information to the patient;
c) determining (130) by the processing unit if the patient has not reached a required position due to a physical or cognitive limitation, the determining comprising utilising the body positioning information and the position and movement image data of the patient, wherein a determination is made that the patient has not reached the required position due to a physical limitation based on a determination that the patient has moved in a correct direction, and wherein a determination is made that the patient has not reached the required position due to a cognitive limitation based on a determination that the patient has moved in an incorrect direction or has not undertaken a substantive movement;
wherein the method comprises either:
adapting by the processing unit the body positioning information in at least one first manner based on a determination that the patient has not reached the required position due to a physical limitation; or
adapting by the processing unit the body positioning information in at least one second manner based on a determination that the patient has not reached the required position due to a cognitive limitation; and
d) after adapting the body positioning information acquiring (140) by a medical image acquisition unit the medical image of the patient.

15. A computer program element for controlling a system according to any one of claims 1 to 13, which when executed by a processor is configured to carry out the method of claim 14.
